Europäisches Patentamt

European Patent Office

Office européen des brevets

(11) Publication number: **0 082 691**
**B1**

(12) **EUROPEAN PATENT SPECIFICATION**

(45) Date of publication of patent specification: **16.04.86**

(21) Application number: **82306751.7**

(22) Date of filing: **17.12.82**

(51) Int. Cl.⁴: **G 02 B 6/04, G 02 B 19/00**

(54) **An illuminating device having optical light guide formed as fibre bundle.**

(30) Priority: **18.12.81 JP 203637/81**

(43) Date of publication of application:
**29.06.83 Bulletin 83/26**

(45) Publication of the grant of the patent:
**16.04.86 Bulletin 86/16**

(84) Designated Contracting States:
**AT BE CH DE FR GB IT LI NL SE**

(56) References cited:
**DE-B-1 224 528**
**US-A-3 926 501**
**US-A-4 294 511**

(73) Proprietor: **OLYMPUS OPTICAL CO., LTD.**
**43-2, 2-chome, Hatagaya Shibuya-ku**
**Tokyo 151 (JP)**

(72) Inventor: **Takami, Akiyoshi**
**446, Nakakayama Hidaka-Cho**
**Irima-Gun Saitama-Ken (JP)**

(74) Representative: **Jones, Colin et al**
**W.P. THOMPSON & CO. Coopers Building**
**Church Street**
**Liverpool L1 3AB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an illuminating device having an optical light guide formed as a fibre bundle and which may be used, for example, in an endoscope, and more particularly to an illuminating device which includes a light source, an optical light guide formed as a fibre bundle for conducting illuminating light from the light source to an object to be illuminated, and a light source lens for directing said illuminating light from the light source to said fibre bundle.

Generally, optical light guide fibre is made from glass or plastics material by crucible, acid melting method, rod, or thermal fusion method. These fibres are bundled, end rings are fitted at the two ends of the bundle, and both ends are suitably ground to form a fibre bundle which is flexible, semi-rigid or rigid.

Such fibre bundle is used in light communication, a light sensor head, light image transport, an illuminating device or a safety anti-explosion device. In any case, it is important to obtain a low loss and good light conducting fibre bundle.

One proposal to solve the problem is described in Japanese Laid-Open Patent Application No. 19761/1979. The proposal described in this application aims to decrease Fresnel reflection on both input and output end surfaces of the fibre bundle and describes that a reflection decreasing layer e.g. MgF$_2$ may be applied on a glass base plate having substantially the same refractive index as that of the fibre bundle and the glass base plates are adhered to input and output end surfaces of the fibre bundle by transparent adhesive also having substantially same refractive index as that of the fibre bundle.

Such proposal only decreases Fresnel reflection loss on the two end surfaces of the fibre bundle, and there is no description of a method of uniformly distributing input light to the input surface of the fibre bundle.

In the art of endoscopes, high accuracy and high capacity are desired and especially a high light conduction property is desired. In conventional endoscopes, to improve light conduction, a strong light source, e.g. small xenon lamp of high luminance is used and also a condenser lens is inserted to concentrate the high output of the lamp on the narrow end surface of the fibre bundle efficiently.

However, such device may cause other problems, such as service life of the lamp and burning of the end surface of the fibre bundle, by the highly concentrated high luminance light. Consequently, a most important problem of such an illuminating device is to concentrate the illuminating light from the light source uniformly and with least light loss to the incident end surface of the fibre bundle.

Accordingly, one object of the present invention is to provide an illuminating device having an optical light guide formed as a fibre bundle in which illuminating light from light source is distributed substantially uniformly over the whole of the incident end surface of the fibre bundle.

Another object of the present invention is to provide an illuminating device having an optical light guide formed as a fibre bundle in which illuminating light from the light source is applied substantially uniformly and with least light loss to substantially the whole of the incident end surface of the fibre bundle.

Thus the invention provides an illuminating device including a light source, an optical light guide formed as a fibre bundle for conducting illuminating light from the light source to an object to be illuminated, and a light source lens for directing said illuminating light from the light source to said fibre bundle, characterized in that an optical reflecting body made of transparent optical material and having a peripheral reflecting portion is mounted on the incident end surface of said fibre bundle and in that said lens and said optical reflecting body are determined to satisfy the formula:

$$l + (-S + \frac{d}{n}) \tan\alpha \geqq \frac{1}{3} r$$

in which:
l=height of bright spot image,
S=air equivalent length between bright spot image and incident end surface of the optical reflecting body and is positive when measured in a direction from the incident end surface towards the fibre bundle,
d=length of the optical reflecting body,
n=refractive index of the optical reflecting body,
α=angle of marginal ray relative to the optical axis, and
r=effective radius of the fibre bundle.

One embodiment of the invention is characterised in that it meets the additional condition:

$$h < \overline{r}$$

in which:
h=height of upper side ray on the incident end surface of the optical reflecting body, and
$\overline{r}$=radius of the optical reflecting body.

The illuminating device according to the present invention distributes illuminating light from the light source substantially uniformly to substantially all the incident end surface of the fibre bundle with least light loss. Thus, the transport efficiency of light is improved and too narrow a concentration of light on the incident end surface of fibre bundle is avoided.

The invention is further described, by way of example with reference to the accompanying drawings, in which:—

Figure 1 is a schematic side view of a known illuminating device utilizing a fibre bundle,

Figure 2 is a schematic side view of a portion of another known illuminating device with a schematic beam path,

Figure 3 is a schematic side view of an illuminating device, according to the present invention,

Figure 4 is a graph of light intensity distribution of bright spot image which is substantially flat, and

Figure 5 is a larger scale view of a front portion of the device of Fig. 3 and shows a beam path corresponding to the light intensity distribution of bright spot image shown in Fig. 4.

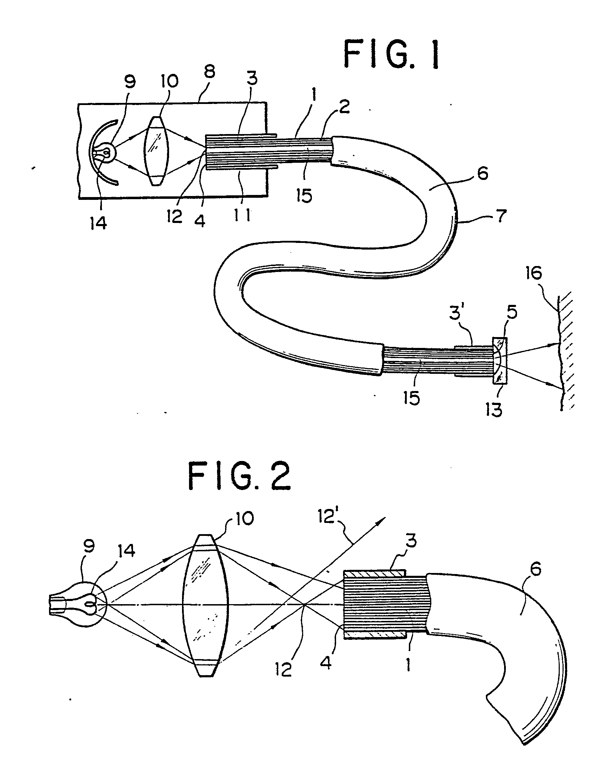

Before particularly describing the present invention, a known illuminating device utilising an optical light guide formed as fibre bundle will be explained referring to Fig. 1.

In Fig. 1 a fibre bundle 1 for illumination is made of a bundle of glass fibres 2 of e.g. tens to thousands of fibres. Rings 3 and 3' are fitted at both ends of the bundle, and a thermo-setting resin adheres between the fibres 2 and also between the rings 3 and 3' and the bundle 1. The incident end surface 4 and emergent end surface 5 of the fibre bundle 1 are ground flat. A flexible portion 6 between the two ends 4 and 5 of the bundle 1 is covered by a cover tube 7, e.g. a silicone tube.

A light source device 8 comprises a special lamp 9 e.g. a halogen or xenon lamp, a condenser lens 10 and a connector 11. When the lamp 9 is lit a bright spot image 12 produced by the lens 10 is formed on the incident end surface 4 of the fibre bundle 1 to perform effective incidence of illuminating light from the light source. On the emergent end surface 5 of the fibre bundle 1, an illuminating lens 13 is mounted to diverge illuminating light which is transmitted through the bundle 1 of the glass fibres 2.

In the above described illuminating device utilizing an optical light guide formed as a fibre bundle, the incident end surface 4 of the fibre bundle 1 is inserted into the connector 11 of the light source device 8, and the lamp 9 is lit so that the bright spot image 12 of the filament 14 of the lamp 9 is formed on the incident end surface 4 of the fibre bundle 1. When the dimension of the bright spot image 12 of the filament 14 is smaller than the effective diameter ($\phi D$) of the incident end surface 4 of the fibre bundle 1, a portion of the incident end surface 4 is illuminated more strongly than other portions. In the fibre bundle 1, which is intended for illuminating purposes, the arrangement of each fibre 2 at the incident end surface 4 and the emergent end surface 5 does not exactly correspond compared with the image transporting fibre bundle. However, fibres tend to gather to form multi-fibres 15 shown in Fig. 1, so that correspondence exists between the incident end surface and the emergent end surface. Thus, generally speaking, the arrangement of each fibre at the incident end surface 4 and the emergent end surface correspond to each other even for illuminating purpose light guides. Thus, when the bright spot image 12 of the light source 9 is formed on a portion of the incident end surface 4 of the fibre bundle 1, e.g. on the multi-fibres 15, the bright spot image 12 is also formed on the emergent end surface 5 without scattering over a wide range. The illuminating light from the end surface 5 is diverged within a narrow range of a body 16 to be observed. Such illuminating light distribution is not desired for observance of a body cavity using an endoscope.

To mitigate such disadvantage, the incident end surface of the fibre bundle 1 is separated a few millimetres from the image point of the bright spot image 12 as shown in Fig. 2. Thus, the bright spot image on the incident end surface 4 becomes blurred to distribute illuminating light all over the end surface 4. However, as the bright spot is not now a sharp spot, light rays 12' on the periphery of the bright spot miss the incident end surface 4 of the fibre bundle 1. Thus, uneven distribution at the incident end point is avoided, however, the amount of light utilized is decreased and light transport efficiency is reduced.

The preferred embodiments of the invention will now be described.

In all figures of the drawings, the same reference numerals denote the same or a similar part or portion.

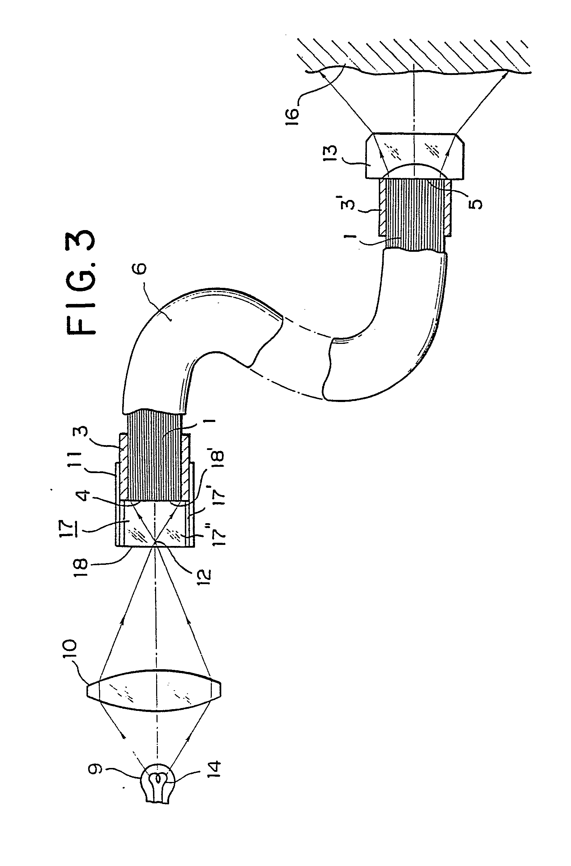

A first embodiment to explain basic construction of the present invention is shown in Fig. 3. A single light guide 17 is made of a core 17'' and a cladding 17' which is secured to the outside surface of the core 17''. The single light guide 17 acts as an optical reflecting body and is mounted in front of the incident end surface 4 of the fibre bundle 1. When the single light guide 17 is connected to the fibre bundle 1, the bright spot image 12 of the light source 9 focuses in the single light guide 17 and diverges and then falls on to the end surface 4 of the fibre bundle 1. Thus, the peripheral rays forming the bright spot also fall onto the end surface 4 of the fibre bundle 1 by reflection at the peripheral wall surface of the single light guide 17.

By connecting the single light guide 17 to the incident end surface 4 of the fibre bundle 1, illuminating light from the light source 9 can be applied to the incident end surface 4 without causing light loss. However, to improve light transport efficiency, the dimensions and refractive index of the single light guide 17 and imaging point of the bright spot image or the air equivalent length of the image point from the incident end surface 18 of the single light guide 17, must be selected accurately and determined.

The selection of the parameters of the single light guide 17 will be explained.

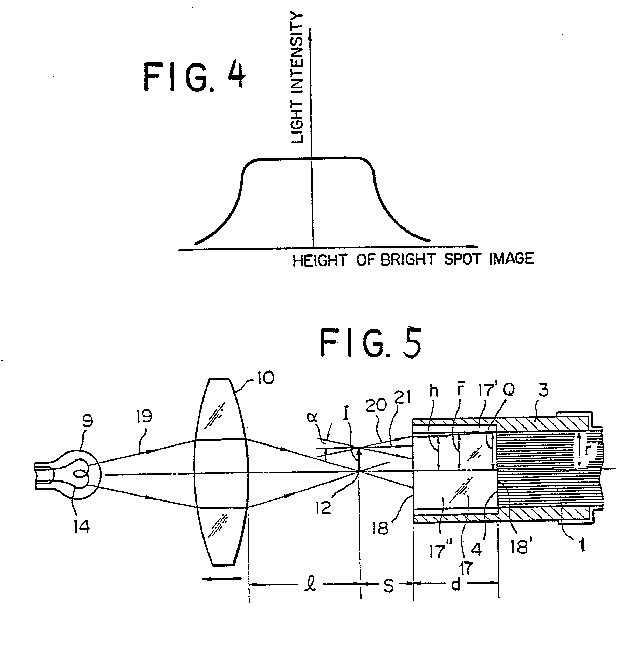

It is assumed that the light intensity distribution of the bright spot image 12 of the light source is substantially uniform, as shown if Fig. 4.

In a fibre bundle connected to a single light guide 17, parameters as shown in Fig. 5 to obtain suitable conditions for incident illuminating light to fall uniformly on the incident end surface without causing light quantity loss are defined as follows:—

    d: length of the single light guide 17,
    $\overline{r}$: radius of the core 17'' of the light guide 17,
    n: refractive index of the core 17'',

r: effective radius of the fibre bundle 1;

l: height of the bright spot image,

S: equivalent air length from the incident end surface 18 of the single light guide 17 to the bright spot image, and is positive when measured in the direction from the end surface 18 towards the fibre bundle,

h: height of upper light ray at incident end surface 18 of the single light guide 17,

Q: height of upper ray 20 where it intersects the incident end surface 4 of the fibre bundle 1,

$\alpha$: angle of the marginal ray 19 to the optical axis.

Generally, height $l$ of the bright spot image is small compared with distance $l$ between the light source lens 10 and the image point of the bright spot image 12, and it can be considered that the principal ray 21 is substantially parallel to the optical axis. In this case, the light stop is considered to be the lens frame. To improve illumination light distribution on the incident end surface 4 and taking into account the random distribution in the fibre bundle 1, Q may in practice be larger than 1/3 of effective radius $r$ of the fibre bundle 1.

Thus,

$$Q \geqq \frac{1}{3} r \qquad (1)$$

But;

$$Q = l + (-S + \frac{d}{n}) \tan\alpha \qquad (2)$$

From formulae (1) and (2), the condition to obtain improved illumination light distribution on the incident end surface 4 is:

$$l + (-S + \frac{d}{n}) \tan\alpha \geqq \frac{1}{3} r \qquad (3)$$

The condition to avoid light loss on the incident end surface 18 of the single light guide 17 is that the upper ray 20 is within the single light guide 17.

Thus,

$$h < \overline{r} \qquad (4)$$

Accordingly, on the assumption that the light intensity distribution of the bright spot image is substantially uniform as shwon in Fig. 4, when parameters l, S, d, n, and $\alpha$ relating to the dimension of the single light guide 17 and image point of the bright spot image, are selected to satisfy formula (3), an illuminating device utilizing a fibre bundle is obtained to direct illuminating light substantially uniformly on to the incident end surface 4 of the fibre bundle 1. Also, when the parameters l, S, d, n, $\alpha$, h and $\overline{r}$ are selected to satisfy formulae (3) and (4), an illuminating device, utilizing a fibre bundle connected to the single light guide, is obtained to direct illuminating light substantially uniformly on the incident end surface 4 of the fibre bundle without causing light loss.

When light flux is not symmetrical about the optical axis by, e.g. the insertion of light stop, the values of h, k, and $\alpha$ in the equations (2) and (3) should be the minimum values respectively.

The radius $\overline{r}$ of the core 17'' of the single light guide 17 may preferably be determined from the manufacturing stand-point such that the core radius $\overline{r}$ of the single light guide 17 is larger than the effective radius r of the fibre bundle 1, so as to compensate for any misalignment between the single light guide 17 and the incident end surface 4 of the fibre bundle 1. Thus, the effective area of the fibre bundle 1 can be fully utilized to transport illuminating light.

The numerical aperture of the single light guide 17 may be the same as or more than that of the fibre bundle 1 so that a maximum angular component of light can be transported to the incident end surface 4 of the fibre bundle 1.

In an illuminating device utilizing a light transporting fibre bundle 1, the single light guide 17 is connected to the incident end surface 4 of the fibre bundle 1, and various parameters of the component parts can be determined by the above described formulae according to the present invention. In practice, component parts can be easily made in conformity with the above-mentioned formulae. However, on assembly, when the fibre bundle 1 is connected to the connector 11 of the light source device 8, some misalignment can occur. To compensate for such misalignment, the condenser lens 10 of the light source device 8 is axially adjustable to adjust the image point and size of the bright spot image. Thus, some parameters can be adjusted. Consequently, illuminating light falls on the incident end surface 4 of the fibre bundle 1 most efficiently with minimum loss and to the full effective area of the end surface.

In the above-mentioned illuminating device, when Fresnel reflection loss is decreased, light transport characteristics are improved. To decrease the Fresnel reflection loss, both end surfaces 18 and 18' of the single light guide 17 and the incident end surface 4 of the fibre bundle 1 are coated with a thin layer of material having a similar or the same refractive index as that of the core 17'' of the single light guide 17, e.g. magnesium fluoride or silicon oxide. Adhesive applied between the incident end surface 4 of the fibre bundle 1 and the single light guide 17 may also be a material having a similar or the same refractive index as that of the core 17'' of the single light guide 17. Such material may be a thermo-setting resin or organic silicone compound of transparent, relatively high heat resistivity, and slightly lower hardness than the core, when cured. Such thin layer coating and applied adhesive decrease end surface reflection loss and also act to waterproof the end surfaces.

In the above-mentioned embodiment, the light reflection body which is connected to the incident end surface 4 of the fibre bundle 1 is a cladded or sheathed single light guide 17. However, a cylindrical reflection body or conical reflection body which reflects light at its peripheral wall surface may be used in place of the single light guide 17. Of course, the optical reflecting body 17 is made from transparent material to assure a good transmission factor.

**Claims**

1. An illuminating device including a light source (9), and optical light guide formed as a fibre bundle (1) for conducting illuminating light from the light source (9) to an object to be illuminated, and a light source lens (10) for directing said illuminating light from the light source (9) to said fibre bundle (1), characterized in that an optical reflecting body (17) made of transparent optical material (17'') and having a peripheral reflecting portion is mounted on the incident end surface (4) of said fibre bundle (1), and in that said lens (10) and said optical reflecting body (17) are determined to satisfy the formula:

$$I+(-S+\frac{d}{n})\tan\alpha\geqq\frac{1}{3}r$$

in which:
I=height of bright spot image (12),
S=air equivalent length between bright spot image (12) and incident end surface (18) of the optical reflecting body (17) and is positive when measured in a direction from the incident end surface (18) towards the fibre bundle (1),
d=length of the optical reflecting body (17),
n=refractive index of the optical reflecting body (17),
α=angle of marginal ray (19) relative to the optical axis, and
r=effective radius of the fibre bundle (1).

2. An illuminating device according to claim 1, characterised in that it meets the additional condition:

$$h<\overline{r}$$

in which:
h=height of upper side ray (20) on the incident end surface (18) of the optical reflecting body (17), and
$\overline{r}$=radius of the optical reflecting body (17).

3. An illuminating device according to claim 1 or 2, in which said light source lens (10) is axially displaceably mounted relative to the light source (14) to regulate the bright spot image.

4. An illuminating device according to any of claims 1 to 3, in which the numerical aperture of the optical reflecting body (17) is the same as or greater than that of said fibre bundle (1).

5. An illuminating device according to any of claims 1 to 3, in which the diameter of the optical reflecting body (17) is greater than the effective diameter of said fibre bundle (1).

6. An illuminating device according to any preceding claim, in which said optical reflecting body (17) and said fibre bundle (1) are adhered to one another by an adhesive having the same or a similar refractive index as that of said optical reflecting body.

7. An illuminating device according to any preceding claim, in which both end surfaces of the optical reflecting body and the incident end surface of the fibre bundle are coated with a reflection-decreasing layer having the same or a similar refractive index as that of the optical reflecting body.

**Patentansprüche**

1. Beleuchtungsvorrichtung mit einer Lichtquelle (9), einem als Fiberbündel (1) ausgebildeten optischen Lichtleiter zum Leiten von Beleuchtungslicht von der Lichtquelle (9) auf einen zu beleuchtenden Gegenstand und einer Lichtquellenlinse (10) zum Richten des Beleuchtungslichtes von der Lichtquelle (9) auf das Fiberbündel (1), dadurch gekennzeichnet, daß ein optischer reflektierender Körper (17) aus transparentem optischen Material (17'') mit einem reflektierenden Umfangsabschnitt an der Einfallsendeoberfläche (4) des Fiberbündels (1) angebracht ist, und daß die Linse (10) und der optische reflektierende Körper (17) zur Erfüllung der folgenden Formel ausgebildet sind:

$$I+(-S+\frac{d}{n})\tan\alpha\geqq\frac{1}{3}r$$

in der:
I die Höhe des Bildes (12) des Hellpunktes,
S die Luftäquivalentlänge zwischen dem Bild (12) des Hellpunktes und der Einfallsendeoberfläche (18) des optischen reflektierenden Körpers (17), und zwar positiv, wenn in einer Richtung von der Einfallsendeoberfläche (18) in Richtung auf das Fiberbündel (1) gemessen,
d die Länge des optischen reflektierenden Körpers (17),
n der Brechungsindex des optischen reflektierenden Körpers (17),
α der Winkel des Randstrahles (19) gegenüber der optischen Achse, und
r der wirksame Radius des Fiberbündels (1) ist.

2. Beleuchtungsvorrichtung nach Anspruch 1, dadurch gekennzeichnet, daß sie die zusätzliche Bedingung:

$$h<\overline{r}$$

erfüllt, in der h die Höhe des oberen Seitenstrahles (20) auf der Einfallsendeoberfläche (18) des optischen reflektierenden Körpers (17) und $\overline{r}$ der Radius des optischen reflektierenden Körpers (17) ist.

3. Beleuchtungsgerät nach Anspruch 1 oder 2, bei dem die Lichtquellenlinse (10) gegenüber der Lichtquelle (14) axial verlagerbar angeordnet ist, um das Hellpunktbild zu regulieren.

4. Beleuchtungsvorrichtung nach einem der Ansprüche 1 bis 3, bei der die numerische Apertur des optischen reflektierenden Körpers (17) gleich wie oder größer als die des Fiberbündels (1) ist.

5. Beleuchtungsvorrichtung nach einem der Ansprüche 1 bis 3, bei der der Durchmesser des optischen reflektierenden Körpers (17) größer als der wirksame Durchmesser des Fiberbündels (1) ist.

6. Beleuchtungsvorrichtung nach einem vorhergehenden Anspruch, bei der der optische reflektierende Körper (17) und das Fiberbündel (1) aneinander mit Hilfe eines Klebers befestigt werden, der den gleichen oder einen ähnlichen Brechungsindex aufweist wie der optische reflektierende Körper.

7. Beleuchtungsvorrichtung nach einem vorhergehenden Anspruch, bei der beide Endoberflächen des optischen reflektierenden Körpers und die Einfallsendeoberfläche des Fiberbündels mit einer reflektionsvermindernden Schicht beschichtet sind, die den gleichen oder einen ähnlichen Brechungsindex aufweist wie der optische reflektierende Körper.

**Revendications**

1. Un dispositif d'éclairage comportant une source lumineuse, un guide optique de lumière sous la forme d'un faisceau (1) de fibres pour transmettre la lumière d'éclairage de la source lumineuse (9) à un objet à éclairer, et une lentille (10) de source lumineuse pour diriger ladite lumière d'éclairage de la source lumineuse (9) vers ledit faisceau (1) de fibres, caractérisé en ce qu'un corps de réflexion optique (17) fait d'un matériau optiquement transparent (17'') et ayant une partie périphérique réfléchissante est monté sur la surface d'extrémité d'incidence (4) dudit faisceau (1) de fibres, et en ce que ladite lentille (10) et ledit corps de réflexion optique (17) sont déterminés pour satisfaire à la formule:

$$l+(-S+\frac{d}{n})\tan\gtreqless\frac{1}{3}r$$

dans lequel:

l=hauteur de la tache image lumineuse (12),
S=distance équivalente dans l'air entre la tache image lumineuse (12) et la surface d'extrémité d'incidence (18) du corps de réflexion optique (17) et qui est comptée positivement lorsqu'elle est mesurée dans la direction de la surface d'extrémité d'incidence (18) vers le faisceau de fibres (1),
d=longueur du corps de réflexion optique (17)
n=indice de réfraction du corps de réflexion optique (17),
α=angle du rayon périphérique (19) par rapport à l'axe optique et,
r=rayon effectif du faisceau (1) de fibres.

2. Un dispositif d'éclairage selon la revendication 1, caractérisé en ce qu'il remplit la condition supplémentaire:

$$h<\overline{r}$$

dans laquelle:

h=niveau du rayon latéral supérieur (20) par rapport à la surface d'extrémité d'incidence (18) du corps de réflexion optique (17), et
$\overline{r}$=rayon du corps de réflexion optique (17).

3. Un dispositif d'éclairage selon la revendication 1 ou 2, dans lequel ladite lentille 10 de source lumineuse est montée mobile axialement par rapport à la source lumineuse (14) pour régler la tache image lumineuse.

4. Un dispositif d'éclairage selon l'une quelconque des revendications 1 à 3, dans lequel l'ouverture numérique du corps de réflexion optique (17) est identique ou supérieure à celle dudit faisceau (1) de fibres.

5. Un dispositif d'éclairage selon l'une quelconque des revendications 1 à 3, dans lequel le diamètre du corps de réflexion optique (17) est supérieur au diamètre effectif dudit faisceau (1) de fibres.

6. Un dispositif d'éclairage selon l'une quelconque des revendications précédentes, dans lequel ledit corps de réflexion optique (17) et ledit faisceau (1) de fibres sont collés l'un à l'autre au moyen d'un adhésif ayant un indice de réfraction identique ou similaire à celui dudit corps de réflexion optique.

7. Un dispositif d'éclairage selon l'une quelconque des revendications précédentes, dans lequel les deux surfaces d'extrémité du corps de réflexion optique et la surface d'extrémité incidente du faisceau de fibres sont revêtues d'une couche de diminution des réflexions ayant un indice de réfraction identique ou similaire a celui dudit corps de réflexion optique.

# FIG. 1

# FIG. 2

FIG.3

0 082 691

# FIG. 4

LIGHT INTENSITY

HEIGHT OF BRIGHT SPOT IMAGE

# FIG. 5